# EUROPEAN PATENT SPECIFICATION

(11) **EP 3 409 275 B1**
(45) Date of publication and mention of the grant of the patent: **28.06.2023**
(21) Application number: 18175296.5
(22) Date of filing: 31.05.2018
(51) Int. Cl.: A61K 31/164, A61K 31/12, A61K 31/4525, A61P 29/02, A61P 29/00, A61K 9/20, A61K 36/9066, A61K 36/67, A61K 9/00, A61K 9/06, A61K 9/08, A61K 9/107, A61K 9/14, A61K 9/16, A61K 9/48

(54) **COMPOSITION FOR PREVENTION AND/OR TREATMENT OF DISEASES ASSOCIATED TO HYPERALGESIA**
ZUSAMMENSETZUNG ZUR PRÄVENTION UND/ODER BEHANDLUNG VON HYPERALGESIE-ASSOZIERTEN ERKRANKUNGEN
COMPOSITION DESTINÉE À LA PRÉVENTION ET/OU LE TRAITEMENT DES MALADIES ASSOCIÉES À L'HYPERALGÉSIE

(30) Priority: 01.06.2017 IT 201700060506
(43) Date of publication of application: 05.12.2018
(73) Proprietor: Neilos S.r.l., 80063 Piano di Sorrento (NA) (IT)
(72) Inventor: DI MAIO, Umberto, 80063 Piano di Sorrento NA (IT)
(74) Representative: Di Giovine, Paolo

(56) References cited:
- EP-A1- 3 130 336
- WO-A1-2016/183134
- LI QIUPING ET AL: "Curcumin-piperine mixtures in self-microemulsifying drug delivery system for ulcerative colitis the", INTERNATIONAL JOURNAL OF PHARMACEUTICS, ELSEVIER, AMSTERDAM, NL, vol. 490, no. 1, 6 May 2015 (2015-05-06), pages 22-31, XP029179830, ISSN: 0378-5173, DOI: 10.1016/J.IJPHARM.2015.05.008
- PANAHI YUNES ET AL: "Antioxidant and anti-inflammatory effects of curcuminoid-piperine combination in subjects with metabolic syndrome: A randomized controlled trial and an updated meta-analysis", CLINICAL NUTRITION, CHURCHILL LIVINGSTONE, LONDON, GB, vol. 34, no. 6, 7 January 2015 (2015-01-07), pages 1101-1108, XP029324349, ISSN: 0261-5614, DOI: 10.1016/J.CLNU.2014.12.019
- Stefania Petrosino ET AL: "Themed Section: Principles of Pharmacological Research of Nutraceuticals", British Journal of Pharmacology British Journal of Pharmacology, 1 January 2017 (2017-01-01), pages 1349-1365, XP055450423, Retrieved from the Internet: URL:http://onlinelibrary.wiley.com/store/1 0.1111/bph.13580/asset/bph13580.pdf?v=1&t= jdkcfxu3&s=990480fe921c27074d45908010f6373 b497f8407
- EUN YEONG LIM ET AL: "Food-Derived Natural Compounds for Pain Relief in Neuropathic Pain", BIOMED RESEARCH INTERNATIONAL, vol. 2016, 1 January 2016 (2016-01-01), pages 1-12, XP055450350, ISSN: 2314-6133, DOI: 10.1155/2016/7917528

## Description

### FIELD OF THE INVENTION

The present invention relates to a composition comprising palmitoylethanolamide, curcumin or a plant extract belonging to genus Curcuma comprising curcumin, and piperine or a plant extract belonging to the Piperaceae family comprising piperine for use in the treatment of hyperalgesia and of diseases associated thereto.

### STATE OF ART

The inflammatory processes are very widespread and associated to a high number of diseases. The drugs currently available for prevention or treatment of inflammation have different disadvantages in terms of toxicity and side effects. Therefore the problem is much felt of providing new compositions not having these disadvantages. Moreover, the problem is felt of providing new compositions which, apart from having a beneficial effect on the inflammatory processes, at the same time have also an analgesic effect.

Patent application EP 3 130 336 A1 discloses food and/or nutraceutical compositions comprising palmitoylethanolamide (PEA) and a second ingredient (e.g. Curcuma longa) that modifies its release in the organism, for an analgesic, antioxidant and anti-inflammatory effect.

International application WO 2016/183134 A1 discloses compositions comprising PEA in combination with a curcuminioid (e.g. curcumin), for alleviating pain and inflammation.

The object of the present invention is to provide a composition alternative to those known in the state of art for use in the treatment of hyperalgesia.

### SUMMARY OF THE INVENTION

The present invention is based on research and on identification of a new combination of active principles exerting both effects of prevention and reduction in the inflammatory response and an analgesic effect due to the action of each single element of the combination and to the synergistic and strengthened action of the several components of the combination the invention relates to.

The present invention relates to compositions comprising or consisting of a mixture of palmitoylethanolamide, curcumin or a plant extract belonging to genus Curcuma comprising curcumin, and further including piperine or a plant extract belonging to the Piperaceae family comprising piperine. for use in treatment of hyperalgesia and diseases associated thereto. Other advantages and features of the present invention will result evident from the following detailed description.

### DETAILED DESCRIPTION OF THE INVENTION

The invention is defined by the claims. Any subject-matter falling outside the scope of the claims is provided for information only.

The present invention describes a composition comprising as main active ingredients, palmitoylethanolamide, curcumin and piperine. The present invention relates to compositions consisting of mixtures of palmitoylethanolamide, curcumin or a plant extract belonging to genus Curcuma comprising curcumin, piperine or a plant extract belonging to the Piperaceae family comprising piperine for use in the treatment of hyperalgesia.

Palmitoylethanolamide, also known as PEA, N-palmitoylethanolamine, N-(2-hydroxyethyl) hexadecanamide or Palmidrol, is an amide deriving from palmitic acid and ethanolamine. It belongs to the family of N-acylethanolamines, thereto even anandamide, the endogen ligand of the cannabinoid receptors and oleylethanolamide belong. PEA was identified for the first time in the '50s, as anti-inflammatory compound insulated in the egg yolk.

In animals, PEA biosynthesis takes place by means of hydrolysis of its direct phospholipidic precursor, la N-palmitoyl-phosphatidylethanolamine, by the action of Phospholipase D selective for N-acyl-phosphatidylethanolamine. The PEA degradation to palmitic acid and ethanolamine takes place due to the action of two different hydrolytic enzymes, fatty acid amide hydrolase (FAAH) and, more specifically, N-acylethanolamine-hydrolysing acid amidase (NAAA).

Preclinical and clinical studies suggest that PEA could be potentially useful in treatment of several diseases, such as eczema, pain and neuronal degeneration and that, at the same time, it could be have not unwished effects in the human beings. Palmitoylethanolamide showed its effectiveness in a variety of pain models, thereamong hyperalgesia induced by carrageenan and by prostaglandin, in the test of persistent pain induced by formalin, in visceral hyperalgesia produced by NGF (nerve growth factor) instillation in bladder and in the model of neuropathic pain induced by sciatic nerve binding. However, it did not show to be particularly effective in thermal hyperalgesia.

There are different mechanisms explaining PEA pharmacological action. This compound, in fact, can act directly or indirectly on different receptors. PPAR-α receptor is a nuclear receptor belonging to the family of PPARs, the receptors activated by peroxisomal proliferators, proteins capable of transferring in the cellular nucleus, of interacting with DNA and modulating the gene transcription. PPAR-α receptor is expressed in different tissues and organs, such as intestine, heart, liver, kidney, muscle and adipose tissue and in some cells of the immune system. The expression of this protein in the cells of the immune system allows it to modulate the inflammation processes. PEA capability of behaving as a agonist of PPAR-α receptors was demonstrated, as a study of LoVerme et al. demonstrates, which demonstrated the capability of direct activation of the receptor, with EC₅₀ of 3 µM, a power comparable to the one of the compound of synthesis Way-14643, which was capable of producing a marked anti-inflammatory response. It was further assumed that, as all PPAR agonists, the bond of PEA to PPAR-α could induce heterodimerization of this receptor with the receptor of retinoic acid (RXR), by forming a complex which transfers into the nucleus and inhibits the transcription of the pro-inflammatory genes.

GPR55 orphan receptor belongs to the family of GPCR receptors and, although it shows a low structural homology with CB₁ and CB₂ receptors, it could be activated by cannabinoids such as Δ9-tetrahyidrocannabinol and anandamide. Low concentrations of PEA have the capability of activating this receptor, with EC₅₀ of 4 nM.

Recently, this receptor has appeared as target potential for the pharmacological treatment of inflammation. It is expressed in most part of brain areas and in the gastrointestinal system. The mechanism of the anti-inflammatory action of this receptor has been not yet fully explained.

Among the molecular targets thereon PEA acts indirectly, there are the endocannabinoid receptors. CB₁ and CB₂ receptors too belong to the family of the receptors coupled to protein G. In the brain, CB₁ receptor is often expressed in the presynaptic terminals and, due to its localization, its activation involves an inhibition of releasing neurotransmitters. CB₁ receptor is also in the peripheral nervous system and in most part of organs and tissues. CB₂ receptor is widely expressed in cells (monocytes, macrophages, B and T cells, mast cells) and in peripheral organs (spleen, tonsils, thyme, gastro-intestinal tract and skin) which play a key role in the immune processes. Differently from CB₁ receptor, CB₂ receptor is poorly expressed at the level of the central nerve system: then, it is more involved in the inflammatory response. The activation of CB₁ and CB₂ receptors is not direct, on the contrary it is associated to a known entourage mechanism.

TRPV1 channel receptor is a receptor activated by high temperatures, by low values of pH and by chemical compounds such as capsaicin. It is a non-selective ion channel which allows the passage of mono-and bivalent cations. This receptor is mainly expressed at the level of dorsal root ganglia and on the sensory nervous fibres of type Aδ and C. However, they are also expressed at the level of brain neurons, of keratinocytes and other types of cells. 2 mechanisms were proposed, therethrough PEA could act on TRPV1 receptors. The first mechanism relates to the indirect activation of TRPV1 receptor by means of entourage effect. In particular PEA, probably through allosteric effects, has the capability of increasing the activation and desensitization of the receptor by anandamide and 2-arachidonoylglycerol.

The second mechanism is the activation of TRPV1 receptor after PPAR-α activation.

Currently there are few studies relating pharmacokinetics of palmitoylethanolamide. In fact, the values of oral bioavailability (F_{OS}) and distribution apparent values (V_{d}) have not yet been established. In a preclinical study the area under the curve of PEA plasmatic level was determined, after administration in Wistar mice. After oral administration, PEA reached the maximum plasmatic concentration (Cₘₐₓ = 420 ± 132 nm), corresponding to an increase by 20 times in the basal plasmatic levels (21 ± 4 nM) after a period of time of 15 minutes (tₘₐₓ). After 2 hours from administration, PEA concentration returned to levels comparable to the basal one. PEA removal half-life is about 12 minutes. The area under the curve of PEA plasmatic level (AUC₁₋₈ₕ) corresponds to 6525 ± 1372 ng PEA min ml-1, as shown in the study of Vacondio et al. The tissue distribution of palmitoylethanolamide was also studies. In a study of Artamonov et al., the distribution N-[9,10-3H] PEA was evaluated, in Wistar mice weighing 150-200 g, 20 minutes after the administration (dose of about 100 mCi, 3.3×10⁻⁵ mol/100 g of body weight, corresponding approximatively to 100 mg/kg). The authors of the study observed that about 0.95% of administered PEA was administered to the brain, with a heterogeneous distribution in the different portions of the brain. This is surprising for a lipophilic compound and it would suggest a preferential retention in hypothalamus. An explanation of this phenomenon could be a portion of hypothalamus equipped with a particular binding affinity for PEA. Palmitoylethanolamide can be useful in treatment of diseases associated to or deriving from neurodegeneration, from hyperalgesia and inflammation, as well as brain damages deriving from ischemia.

PEA potential protective effect was demonstrated in different preclinical studies, on experimental models of Alzheimer and Parkinson's disease, as well as other neurodegenerative diseases. For example, in a study on Alzheimer murine model, the subcutaneous administration of this compound reduced the behaviour disorders, lepidic peroxidation, induction of iNOS and activation of caspase-3 induced by the intracerebrovascular administration of β-amyloid 25-35.

PEA exerts a protective action even on neurological disorders induced by brain ischemia and by traumatic brain damage. Under these conditions, in fact, it is often observed an increase in the tissue levels of PEA in the central nervous system, since this molecule has the function of protecting the neurons from the damages induced by ischemia and brain trauma.

Numerous studies demonstrated PEA analgesic action. A first study, performed by Calignano et al., demonstrated PEA capability of reducing hyperalgesia induced by formalin. A mechanism based upon the activation of CB₂ receptor was supposed. Another study demonstrated PEA capability of inhibiting hyperalgesia pain of the model of damage by chronic compression (binding of sciatic nerve). Since PEA administration in mice without PPAR-α receptor did not lead to an analgesic effect, it was assumed that this receptor was involved in the analgesic action mechanism of this substance. Different clinical studies demonstrated PEA analgesic action. In the clinical studies providing micronized PEA administration, alone or in combination with stilbene or polydatin, it was demonstrated that micronized PEA administration, at a dose of 300 mg 2 times/die has the capability of reducing the scores associated to pain in diabetic patients suffering from peripheral neuropathies. PEA administration did not lead to unwished effects during treatment. In another clinical study, performed on 610 patients suffering from pain of different etiopathogeneses, it was demonstrated that PEA administration, at a dose of 600 mg 2 times/die is capable of reducing pain both alone and in association with other analgesics. The pain reduction is observed even in absence of concomitant analgesic therapies and no side effects are found. A lately implemented recent meta-analysis demonstrated PEA effectiveness in reducing pain intensity in patients suffering from chronic or neuropathic pain. This study confirmed that the relief of pain induce by PEA is progressive, independent from age and sex and not correlated to the etiopathogenesis of chronic pain.

PEA anti-inflammatory effects would seem to be more associated to its ability of modulating the activation and the degranulation of the mastocytes, action known as ALIA (autacoid local inflammation antagonism) mechanism. The first experimental proof of PEA activity on inflammation was shown in 1996, with the demonstration of the capability of this molecule to reduce the amount of degranulated mastocytes and oedema induced by substance P in the mouse ear. PEA administration is also associated to the reduction in oedema by carrageenan, deriving from the inactivation of mastocytes and from the consequent reduction in all events started by the activation of mastocytes. Afterwards it was demonstrate even the involvement of CB₂ receptor in the anti-inflammatory mechanism of this molecule. Subsequently it was demonstrated that PEA anti-inflammatory action mechanism also derives from the activation of PPAR-α receptor.

PEA anti-inflammatory effects were investigated in several diseases. The effectiveness of this compound was demonstrated in animal models of intestinal chronic inflammatory diseases, ulcerative colitis, angiogenesis associated to inflammation, therefrom the protective effect on the intestinal mucosa is deduced, both in case of inflammation and cancer. The activity was demonstrated even on allergic dermatitis by contact.

The composition could include an amount of PEA between 10 mg and 5000 mg, preferably between 400 and 800 mg.

Curcuma longa is a plant used by thousands of years in the traditional Chinese medicine for treatment of different types of diseases. It is a perennial plant belonging to the family of Zingiberaceae and it is cultivated in India and in South-East Asia. Curcuma longa mainly includes secondary metabolites: curcumin (diferuloylmethane), demethoxycurcumin and bisdemethoxycurcmin.

Curcumin, the existing main secondary metabolite of Curcuma longa, is provided with several pharmacological activities, such as anti-inflammatory, immunomodulatory, antitumoral and neuro protective activity. It is one of the most powerful anti-inflammatory drugs of natural origin. The main molecular mechanisms of inflammation were widely studied. Different enzymes, cytokines, chemokines and polypeptidic hormones were identified as mediators of inflammation. Thereamong we remind COX-2, 5-lipoxygenase, tumour necrosis factor α, interleukins (IL-1, IL-6, IL-17, IL-21, IL-23) and protein MCP-1. Thereamong the tumour necrosis factor α (TNF-α) is one of the main mediators of inflammation.

Studies demonstrate that curcumin is a highly pleiotropic molecule, capable of interacting with several molecular targets involved in inflammation. The action mechanisms of curcumin are several, but the main one consists in the expression modulation of several proteins, thereamong pro-inflammatory cytokines (TNF-α, IL-8, IL-1β, IL-6), apoptotic proteins, NF-kB, COX-2, STAT3, MDA, etc. Curcumin modulates the inflammatory response down-regulating the activity of cyclooxygenase-2, of lipoxygenase and inducible nitric oxide synthase (iNOS). The inhibition of COX-2 and of iNOS, very probably, is obtained thanks to the suppression of activation of the kappa-B (NF-kB) nuclear factor by curcumin. NF-kB is a ubiquitous transcription factor, involved in regulating inflammation, cellular proliferation and carcinogenesis. It is believed that Curcumin can inhibit NF-kB activation by locking the phosphorylation of the IKB kinase inhibitory factor. Another potential mechanism of the anti-inflammatory action of curcumin consists in regulating AP-1 transcription factor in macrophages.

Animal studies showed that curcumin is metabolized quickly, conjugated in liver and excreted with faeces, therefore it is associated to a limited system bioavailability. Data on pharmacokinetics of Curcumin in man are limited and mainly derive from clinical studies performed on patients with cancer. In a clinical study of phase I, performed on 25 patients with different precancerous lesions, demonstrated that the doses of 4, 6 and 8 g/die of curcumin for a period of 3 months lead to serum concentrations of 0.51 ± 0.11, 0.63 ± 0.06 and 1.77 ± 1.87 µM respectively, demonstrating that oral bioavailability of this compound is very limited. Even the systemic clearance is high and half-life is very low.

Several studies evaluated the anti-inflammatory effects of Curcumin. In a model of oedema by carrageenan in mice, the anti-inflammatory effect of doses of curcumin between 50 and 200 mg/kg was demonstrated. Curcumin is also capable of inhibiting arthritis induced by formaldehyde, in mice, at a dose of 40 mg/kg, which is also associated to a lower ulcerogenic index than that of phenylbutazone at similar doses (0.60 vs 1.70). In a study a dose of 50 mg/kg of curcumin was administered for 10 days before inducing ulcerative colitis by 1,4,6-trinitrobenzenesulphonate: the pre-treatment with this compound lead to a statistically significative reduction in diarrhoea, to an improvement of colon architecture, as well as to reduction in infiltration of neutrophils and lipid peroxidation. Other preclinical studies were performed on modes of Rheumatoid arthritis, pancreatitis and cancer.

The anti-inflammatory action of curcumin was evaluated even in different clinical studies. In a clinical study, performed on 45 patients suffering from post-surgical oedema, the patients were divided into 3 groups, treated respectively with 400 mg of curcumin, lactose (a placebo) or 100 mg of phenylbutazone 3 times/day for 3 days. Curcumin demonstrated to be better than phenylbutazone, thanks to its capability of reducing all inflammation parameters. In another clinical study, the administration of 1200 mg of Curcumin per day demonstrated to be effective in improving the symptoms of rheumatoid arthritis, the swelling of articulations, morning rigidity and deambulation time. In different clinical studies curcumin was used for treating intestinal chronic inflammatory diseases (IBD), in particular Crohn disease and ulcerative colitis. A pilot study was made on patients with IBD previously treated with drugs for ulcerative colitis or *Crohn* disease. 5 patients with proctitis received 550 mg of curcumin 2 times/die for one month and 3 times/die for another month. Other 5 patients, suffering from *Crohn* disease, were treated with 360 mg of curcumin 3 times/die for one month and 4 times/die for the subsequent month. In both groups improvements in the inflammatory indexes in treated patients were observed.

Other clinical studies about the effectiveness of curcumin were performed on patients suffering from osteoarthritis, uveitis, dyspepsia and gastric ulcer, irritable bowel syndrome, pancreatitis and cancer.

Although the main action mechanism of Curcumin is the anti-inflammatory one, other important pharmacological effects are not missing, such as anti-oxidant and hypolipidemic effects.

Curcumin, preferably extracted from *Curcuma longa L.* could be present in the composition in an amount between 10 mg and 10000 mg, preferably between 200 and 800 mg. The composition of the invention then could include even an extract of plants belonging to genus Curcuma, in particular belonging to the species *Curcuma longa.* For example in the composition an extract of Curcuma longa could be present. Such extract will include the active principle curcumin titrated for example in the range comprised between 1% and 99% by weight.

Piperine is a chemical compound belonging to the group of "vanilloids", provided with a molecular portion which can be overlapped to that of vanillin. It is the main alkaloid of black pepper (Piper nigrum L.) and of other plants belonging to the Piperaceae family, provided with pungent taste, due to its capability of activating TRPV1 receptor. It is a secondary metabolite, the biosynthesis thereof provides the reaction between L-lysine and cinnamoyl-CoA.

Bioavailability studies, implemented on mice, showed that piperine is quickly absorbed through the intestinal walls and that the main metabolites, including piperonylic acid, piperonylic alcohol, piperonal and vanillin acid, are excreted mainly by renal route.

Piperine is widely recognized as an *enhancer* of bioavailability, which derives from its capability of inhibiting CYP3A4 cytochrome and glycoprotein-P. This capability of inhibiting the metabolism and outflow of xenobiotics brought to different studies which evaluated the capability of this compound of increasing bioavailability of active principles of natural and synthetic origin.

In these studies, piperine demonstrated to be capable of increasing oral bioavailability of several drugs and nutraceutics, thereamong antioxidants, non-steroidal anti-inflammatory drugs, oral hypoglycaemic drugs, antihistaminics, anti-epileptic drugs, anti-retroviral drugs and other drugs.

Piperine capability of increasing oral absorption of these compounds could have a significant clinical impact in treating disease characterized by high prevalence among population. Bioavailability increasing activity is not the only pharmacological activity of piperine. In fact, it showed an antitumoral, anti-inflammatory and anti-microbial activity.

One of the main problems of curcumin is low oral bioavailability, due to its poor solubility. However, different expedients were found to be able to increase oral bioavailability thereof. One of the most promising methods indeed consists in coadministering curcumin with piperine. This compound, in fact, by inhibiting the conjunction to glucuronic acid of curcumin and its intestinal metabolism, is capable of increasing considerably oral bioavailability thereof. A clinical study demonstrated piperine capability of increasing the plasmatic levels of curcumin from 30 minutes to 1 hour after the administration, with an increase of bioavailability of 2000%. An analogous effect was observed in another clinical study, which provided the administration of 2 g of curcumin on healthy volunteers. The administration of curcumin only has brought to minimum variations in curcumin bioavailability. The co-administration of 2 g of curcumin with 20 mg of piperine brought to decidedly higher plasmatic concentrations of curcumin of 0.25 1 hour after the administration, with an increase in oral bioavailability of 20 times. Piperine could be present in the composition in an amount comprised between 0.01 mg to 500 mg, preferably between 10 and 100 mg. Piperine could be extracted from *Piper nigrum* or other plants of Piper genus or the family of Piperaceae. The composition of the invention then could comprise even a plant extract belonging to the Piperaceae family. For example in the composition an extract of *Piper nigrum* can be present, wherein such extract will include piperine active principle piperine titrated for example in the range between 1% and 99% by weight.

The compositions according to the present invention could be formulated under any administration form and route and associated to any other component, in a variety of ways, preferably for example capsules, soft capsule, tablets, pills, gelatines, powders or granules will be formulated for oral use. Such excipients could be selected among those usually known in the state of art and they include, but they are not limited to: a) carriers, such as for example sodium citrate and calcium phosphate, b) fillers such as example starch, lactose, microcrystalline cellulose, sucrose, glucose, mannitol and colloidal silica, c) moistening agents, such as for example glycerol, d) disintegrating agents, such as alginates, calcium carbonate, starches, starch, cellulose and polyvinyl pyrrolidone derivatives, silicates and sodium carbonate e) binding agents, such as carboxymethylcellulose, alginates, gelatine, polyvinyl pyrrolidone, sucrose, cellulose polymeric derivatives, starch derivatives f) retardant agents such as paraffin, cellulose polymers, esters of fatty acids g) absorption accelerometers, such as quaternary ammonium compounds, h) wetting agents and surfactants such as cetyl alcohol and glycerol monostearate, i) adsorbents, such as benthic clays and kaolin, k) lubricants such as talcum, calcium stearate, magnesium stearate, polyethylene glycol, sodium lauryl sulphate, sodium stearyl fumarate j) glidants such as talcum, colloidal silica.

The forms of solid dosage, such as tablets, capsules, soft capsules, gelatines, pills and granules, could be coated with enteric, gastric coatings or of any other type known in the state of art. They could include opacifiers and they could be of the type to allow the release of the active ingredients only or preferably in a certain tract of intestine, in case in a delayed way. Substances which could allow such delayed use include, but they are not limited thereto, polymers and waxes.

The soft capsules could house the antioxidant active substances in liquid form alone or in solutions, suspensions or emulsions of the active substances in a liquid solvent. The soft capsules could be characterized by a casing qualitative similar to that of the rigid capsules, but thicker and softer.

Liquid forms suitable to an oral administration are for example emulsions, solutions, prepared or extemporary suspensions, syrups and elixirs. Excipients suitable to the formulations according to the present invention in liquid forms for oral use include, without being limited thereto, diluents such as water or other solvents, solubilizing and emulsifying agents selected among ethyl alcohol, polyalcohols, propylene glycol, glycerol, polyethylenglicole and esters of sorbitan. These formulations can even include sweeteners and aromas.

The compositions for example will be a a food supplement, a nutraceutical, dietary and nutritional composition a foodstuff, a beverage, a nutraceutical, a medicament, a medicated food, food for special medical purposes, food. The compositions will be mainly intended to be used by human beings, but they could be even used on animals.

The combination of the above-said active ingredients could be formulated in one single composition according to the various above-described embodiments or in a kit including the different separate ingredients, for example in single compositions such as capsules, pills, tablets for sequential or contemporary administration of the different ingredients.

The above-described compositions could be used/administered/taken for the treatment of hyperalgesia and of diseases associated thereto such as for example osteoarticular and musculoskeletal diseases, headache, neuropathies cystitis, prostatitis, etc. Preferably, independently from the type of used formulation, the combination of active ingredients according to the present invention will be administered with a daily dosage regime according to the above-said concentrations.

Hereinafter a description of inflammatory and hyperalgesia diseases is shown in details.

The inflammatory response which appears at the level of the central and peripheral nervous system is strongly involved in the development and persistence of many painful conditions, through the releasee of inflammatory mediators such as cytokines and prostaglandins. Among the pro-inflammatory cytokines most involved in manifestation of pain and in modulation of neuronal activity, IL-1β, IL-6 and TNF-α are detected. Chemokines are cytokines known for the participation to chemotaxis. These include MIP-1ALFA, MCP-1 and GRO/KC, which result to be up-regulated in neuroinflammation models, of alteration of myelin sheath , in some models of trauma of SNC and of the peripheral nerves. Apart from the cytokines even prostaglandins are involved in the onset of neuropathic pain. Among these ones PGE2 determines the activation of the signal at the level of the microglial cells. The immune response which is triggered in SNC is at the basis of some pathological events such as ischaemias, neurodegenerative diseases and immuno-mediated disorders, apart from being able to contribute to the neuronal damage. Among the neurodegenerative diseases wherein inflammation appears to have an involvement Alzheimer, Parkinson, heart stroke derived by the occlusion of SNC vessels, Amyotrophic Lateral Sclerosis and Multiple Sclerosis are included.

Pain and inflammation characterize several disorders. Cystitis is an inflammation of the low urinary tract, which can appear through dysuria, increase in the urinary frequency, urinary urgency, haematuria and suprapubic pain. Among the most frequent causes of the inflammatory process the infection can be found caused by some bacterial species, especially E. coli. Prostatitis, an inflammation of the prostate, is strongly diffused in urologic field, which can be triggered by infective and not infective causes. Even this condition often is accompanied by pain of the area involved by inflammation.

### HYPERALGESIA

Pain is defined by International Association for the Study of Pain (IASP) as an unpleasant sensorial and emotional experience which is associated to damage or potential tissue damage.

The physiological component of pain is called as "nociception" and it comprises a series of processes for transducing, transmitting and modulating neuronal signals which generate in reply one external stimulus.

The first portion of the process comprises the fact of transducing signals of mechanical, chemical or thermal type into electric pulses through particular nerve terminations known as nociceptors.

The nociceptors are found in any tissue except in the brain thereto they transmit stimuli of different nature. When a nociceptor is activated, the piece of information is transmitted through the nervous axons at first in the trunks (properly called nerves), then in the roots and then to the spinal cord by constituting the spinothalamic tract. From the dorsal horns of the cord the signal is transferred to the brain. The nociceptive pulse is here distributed in different sites by producing a response (pain).

The nociceptors are commonly classified in three categories based upon the type of stimulus thereto they reply and the diameter and speed for conveying the pulse along the axons: Aβ with bigger diameter of the axon (between 6 and 22 microns), are myelinated and capable of conveying the electric pulse very quickly (between 33 and 75 m/s). On the contrary, Aδ nociceptors have intermediate diameter of the axon (between 2 and 5 microns) and the transmission speed between 5 and 30 m/s; at last, the nociceptors of type C have a smaller diameter (0.3-3 microns), they are unmyelinated and they convey the pulse very slowly. It is believed that Aδ and C fibres are those mostly involved in the transportation of the nociceptive signals and in particular they represent the preferential transportation routes for the signals related to sharp pain and chronic pain, respectively.

After a tissue damage of different origin or different localization (inner organs, skin) and the stimulation of the nociceptors, usually potassium ions and several autacoids, such as bradykinin and serotonin, are released. Bradykinin subsequently determines the activation of the nervous fibres and subsequently it activates the inflammatory cascade starting from phospholipase A2 with consequent activation of cyclooxygenase and lipoxygenase with production of prostaglandin, prostacyclin and leukotrienes which amplify the pain sensation. In order to oppose pain sensation, the fibres of the spinal cord, the brain and the peripheral tissues release neuromodulators known as endogenous opioids (dynorphins and β endorphins) which inhibit the action of the neurons involved in the transmission of the pain pulse.

Commonly the pain based upon its duration is classified as sharp pain and chronic pain.

The sharp pain is important for the organism as it represents a wake-up call against a potentially dangerous situation. It can be of somatic nature (coming from the skin or from subcutaneous tissues), visceral nature (inner organs) or referred (felt in a different area with respect to the originally site which determined the nervous stimulus).

The chronic pain generally is characterized by a persistent inflammation (such as arthritis) or by a dysfunction of the nervous system such as in case of neuropathic pain.

In the organism the endocannabinoid system is considered responsible for keeping homoeostasis of different physiological functions such as for example inflammation and pain. This complex system constituted by different ligands, receptors, enzymes involved in biosynthesis and degradation of ligands, explicates its action both at central and peripheral level. In particular, it modulates the neurotransmission at the central level and, at the peripheral level, it explicates its action by preventing degranulation of mastocytes.

The best-known endocannabinoids are anandamide, 2 acyl-glycerol and palmitoylethanolamide (PEA). These bioactive lipids are capable of linking to the receptors for the endocannabinoids such as CB₁ and CB₂ the activation thereof is responsible for different properties such as emetic, antioxidant, immunosuppressive, anti-inflammatory, analgesic, antiepileptic and appetite stimulant properties. These receptors sensible to the endogenous cannabinoids, derived from plants and having synthetic derivation, belong to the family of the receptors coupled to protein G. The receptor activation determines a dose-depending and stereoselective reduction of the activity of adenylate cyclase with consequent lock of cyclic AMP production.

The two receptors are structurally very similar with a similarity of 44%. In the specific case, CB₁ receptors are present abundantly in hippocampus and in the associated cortical regions, in the brain and in the basal ganglia. Such receptor is involved in mitigating the synaptic transmission and partially even in the peripheral analgesic action of endocannabinoids.

On the contrary, CB₂ receptors are most present in T cells, the mastocytes, B lymphocytes and at the level of the hematopoietic cells as well as in the peripheral nervous terminations, playing an important role in the antinociceptive, antalgic and anti-inflammatory activity. In different preclinical models such as those of tactile and thermal allodynia, mechanical and thermal hyperalgesia the reduction in the nociception after activation of CB₂ receptors was demonstrated.

Endocannabinoids of natural origin, thereamong PEA, represent an important alternative to the traditional anti-inflammatory drugs for treatment of different diseases characterized by neurodegeneration, inflammation (neuroinflammation or other types of inflammatory conditions such as pelvic pain, cystitis) and in all diseases characterized by painful symptomatology.

### EXAMPLES

Hereinafter some not limiting examples of daily dosages of the combination of active ingredients used in the compositions of the present invention are shown.

### EXAMPLE 1

| Active principle | Daily dose |
|---|---|
| Palmitoylethanolamide | 400 mg |
| Curcumin | 100 mg |
| Piperine | 20 mg |

### EXAMPLE 2

| Active principle | Daily dose |
|---|---|
| Palmitoylethanolamide | 400 mg |
| Curcumin | 200 mg |
| Piperine | 20 mg |

### EXAMPLE 3

| Active principle | Daily dose |
|---|---|
| Palmitoylethanolamide | 400 mg |
| Curcuma longa L. e.s. | 200 mg |
| Piper nigrum e.s. | 10 mg |

### EXAMPLE 4

| Active principle | Daily dose |
|---|---|
| Palmitoylethanolamide | 400 mg |
| Curcuma longa L. e.s. | 200 mg |
| Piper nigrum e.s. | 20 mg |

### EXAMPLE 5

| Active principle | Daily dose |
|---|---|
| Palmitoylethanolamide | 400 mg |
| Curcuma longa L. e.s. | 400 mg |
| Piper nigrum e.s. | 20 mg |

### EXAMPLE 6

| Active principle | Daily dose |
|---|---|
| Palmitoylethanolamide | 600 mg |
| Curcumin | 200 mg |
| Piperine | 20 mg |

### EXAMPLE 7

| Active principle | Daily dose |
|---|---|
| Palmitoylethanolamide | 800 mg |
| Curcumin | 200 mg |
| Piperine | 20 mg |

### EXAMPLE 8

| Active principle | Daily dose |
|---|---|
| Palmitoylethanolamide | 800 mg |
| Curcumin | 400 mg |
| Piperine | 20 mg |

### EXAMPLE 9 (not according to the invention)

| Active principle | Daily dose |
|---|---|
| Palmitoylethanolamide | 400 mg |
| Curcumin | 400 mg |

### EXAMPLE 10 (not according to the invention)

| Active principle | Daily dose |
|---|---|
| Palmitoylethanolamide | 800 mg |
| Curcumin | 800 mg |

### EXAMPLE 11 Composition

| | |
|---|---|
| Ingredient | Amount |
| Palmitoylethanolamide | 400 mg |
| Curcumin | 200 mg |
| Piperine | 20 mg |
| Microcrystalline cellulose | 300 mg |
| Silicon dioxide | 50 mg |
| Polysorbate 80 | 20 mg |
| Hydroxypropyl cellulose | 15 mg |
| Titanium dioxide | 10 mg |
| Magnesium stearate | 5 mg |

### EXAMPLE 12

The composition of example 11 was prepared by mixing Palmitoylethanolamide, Curcumin and piperine with excipients and then by reducing in form of tablet. The tablet was subsequently coated with a hydroxypropyl cellulose and titanium dioxide-based film.

### Experimental data

### Analgesia and peripheral neuropathy

The neuropathic pain is considered a real pathology therefor currently no effective pharmacological treatments are available. For this reason, the neuropathic pain can be considered a pathology which cannot be treated. This form of pain is supported by activation of glial and microglial cells and often by a complex functional and neuroanatomical remodulation of the spinal and supraspinal neural circuits (pain matrix) involved in pain transmission and integration. The different interventions which are currently performed (pharmacological, physical or psychological/cognitive support) provide a positive result only in a low percentage of subjects (30%), and such value can be overlapped to the one obtained with placebo. Therefore, we believe it fundamental to study pain from a multidisciplinary point of view and above all by experimenting innovative therapies. We believe that the latter are based upon the knowledge of new neurobiological mechanisms and, in particular, in understanding the bases of the glia-neuron functional interaction.

### Objective of experiments

1) Phenotypical characterization of animals subjected to peripheral neuropathy of 2 weeks with interest to the sensorial component (tactile and thermal painful response).
2) Evaluation of the pharmacological effectiveness of the single compounds and of the related combinations in the behaviour alterations induced by neuropathy.
3) Study of the possible molecular and cellular mechanisms at the base of the potential neuroprotective effects of the above-mentioned compounds, by analysing transcription and protein factors in the different spinal and supraspinal areas involved in the mechanisms for inducing pain.

### Treatment scheme

The groups of animals were treated chronically by intraperitoneal route according to the shown scheme and the above-mentioned concentrations:
1. Carrier
2. PEA + CURCUMA
3. CURCUMA + PIPERINE
4. Curcuma+piperine+PEA
5. Sham group control animals

### Induction of neuropathy (SNI)

The neuropathy induced by means of spared nerve injury (SNI) technique is performed by cutting the tibial and peroneal component of the sciatic nerve, by leaving the sural component intact. The animal is anaesthetized, and after monitoring the anaesthesia depth by checking the breech reflex, it is positioned in left side decubitus on a heated examination table to keep constant the bodily temperature, one proceeds with trichotomy of surgical field. The right rear limb is positioned on a small platform with the purpose of keeping it lifted and it is fastened with adhesive tape. On the area to be operated an incision in the side rear portion, above the thigh, is made, by showing the sciatic nerve. The latter originates from L4-L6 spinal segments and it is divided into three branches, the sural nerve, the tibial nerve, the common peroneal nerve and this trifurcation is observed below the biceps femoris muscle. The common peroneal and tibial nerves are insulated from the sural nerve, tied up with a silk thread 5.0, and at a distance between 2-4 mm from tying the cutting is performed: all this by avoiding stretching and the contact with surgical tools of the sural nerve. The wound will be closed with one internal and two external suture stitches (absorbable surgical suture 6.0). The checking animals (sham group) are subjected only to exposure of the nerve and subsequent closure of the cutaneous access.

### Test for evaluating motor deficits

In Rotarod test (Ugo Basile, Varese, Italy) also called rotating roll test, the mouse is positioned on a rotating cylinder and the time (in seconds) is measured wherein it is capable of being balanced before falling. The cylinder appears to be divided by 6 disks into 5 sections, by allowing to subject 5 mice simultaneously to the test, one per section. Therebelow there is a platform, in turn divided into 5 plates (at the 5 sections) each one thereof is connected to a magnet which, activated by the mouse falling on the plate, allows to record its residence time on the cylinder. After an adaptation period of 30 seconds, the rotation speed is gradually increased from 3 to 30 rpm for a maximum period of time of 5 minutes. On the same day the animals are subjected to two tests separated therebetween by a time interval of about one hour. The residence time of the mouse on the cylinder is expressed as latency (sec).

### Test for evaluating the painful behaviour

1) The tactile allodynia is measured by Von Frey. Such method provides the use of numbered filaments (Von Frey hairs) having different thickness, providing an incremental force according to a logarithmic scale. The animals are housed in plastic cages with a floor made of metal grid. After a habituation period of 30 minutes, the filaments are applied with constant force on the plantar surface of the rear paw for 3-5 seconds, with the purpose of determining the response threshold time. The mechanical threshold for withdrawing the paw is quantized by the up-down method, which consists in applying as first filament the one corresponding to 50% of the paw-withdrawal threshold under basal conditions and subsequently a filament with decreasing force or one with increasing force, in presence or absence of nociceptive response, respectively. Each measurement is performed at a distance of at least three minutes from the subsequent one, so as to avoid the presence of amplified responses due to close stimulations.
2) Hyperalgesia is evaluated through Plantar test (Ugo Basile, Varese, Italia). Each mouse is placed in a cage made of plexiglass (22 cm × 17 cm × 14 cm; length × length × height) with a glass bottom. After 1 hour of adaptation the plantar surface of the mouse paw is exposed to a radiant heat beam through the glass bottom (Osram halogen bellaphot bulb; 8 V, 50 W). A photo-electric cell detects the light rejected by the paw and turns-off the beam when the mouse, after the painful sensation, moves the paw by interrupting the reflected light beam. The apparatus measures the time (in seconds) elapsing between the stimulus application and the nociceptive response of the animal. The maximum time of exposition to radiant heat is 10 seconds, so as to prevent possible tissue damages. The nociceptive response of the thermal sensibility is expressed as latency of withdrawing the paw to the thermal stimulus (TWL, Thermal Withdrawal Latency).

At the end of the behaviour tests all animals will be subjected to euthanasia and the organs will be collected with the purpose of processing them for biochemical and immunohistochemistry studies (Western blotting, PCR and Immunofluorescence). Such evaluations will be performed during the course of the pathological state and in presence of pharmacological treatment. In particular, the dosage of cytokines and pro/anti-inflammatory factors and the characterization of specific cellular population (neurons, glia, microglia) will be performed by analysing surface antigens and expression of receptors involved in the neuroinflammatory processes associated to the onset and development of the neuropathic pain.

The results shown in the above-described tests on animals showed the effect obtained by administering the three compounds PEA-CURCUMA-PIPERINE at the same time, the dosages being equal it is significantly higher than the administration of only two active ingredients PEA-CURCUMA or CURCUMA-PIPERINE added to the effect of the third active ingredient alone. In other words, the contemporary use of the three active principles show an unexpected synergic effect on hyperalgesia and the neuropathic pain with respect to the single active ingredients.

## Claims

1. A composition comprising or consisting in a mixture of palmitoylethanolamide, curcumin or a plant extract belonging to genus Curcuma comprising curcumin, and piperine or a plant extract belonging to the *Piperaceae* family comprising piperine, for use in the treatment of hyperalgesia.

2. The composition for use according to claim 1, wherein the composition is for oral use.

3. The composition for use according to claims 1 to 2 wherein the amount of curcumin is between 10 mg and 10000 mg and/or the amount of palmitoylethanolamide is between 10 and 5000 mg.

4. The composition for use according to any one of claims 1 to 3 wherein the amount of piperine is between 0.01 and 500 mg.

5. The composition for use according to any one of claims 1 to 4 in a form selected from capsule, soft capsule, tablet, pill, gelatine, powder, granule, emulsion, solution, syrup.

6. The composition for use according to any one of claims 1 to 5 further comprising one or more carriers and/or excipients.

7. The composition for use according to any one of claims 1 to 6 wherein said composition is a food supplement, a nutraceutical, dietary and nutritional composition, a foodstuff, a beverage, a nutraceutical, a medicament, medicated food or food for special medical purposes.

8. The composition for use according to any one of claims 1 to 7 wherein hyperalgesia is associated to a disease selected from osteoarthritis, neuropathy, headache, migraine, cystitis or prostatitis.

## Patentansprüche

1. Stoffgemisch umfassend oder bestehend aus einem Gemisch von Palmitoylethanolamid, Curcumin oder einem Pflanzenextrakt, der Gattung Curcuma gehört und Curcumin umfasst, und Piperin oder einem Pflanzenextrakt, der zur Familie der *Piperaceae* gehört und Piperin umfasst, zur Verwendung bei der Behandlung von Hyperalgesie.

2. Stoffgemisch zur Verwendung nach Anspruch 1, wobei das Stoffgemisch zur oralen Verwendung bestimmt ist.

3. Stoffgemisch zur Verwendung nach Anspruch 1 oder 2, wobei die Menge an Curcumin zwischen 10 mg und 10000 mg und/oder die Menge an Palmitoylethanolamid zwischen 10 und 5000 mg liegt.

4. Stoffgemisch zur Verwendung nach einem der Ansprüche 1 bis 3, wobei die Menge an Piperin zwischen 0,01 und 500 mg liegt.

5. Stoffgemisch zur Verwendung nach einem der Ansprüche 1 bis 4 in einer Form, ausgewählt aus Kapsel, Weichkapsel, Tablette, Pille, Gelatine, Pulver, Granulat, Emulsion, Lösung, Sirup.

6. Stoffgemisch zur Verwendung nach einem der Ansprüche 1 bis 5 ferner umfassend einen oder mehrere Träger und/oder Hilfsstoffe.

7. Stoffgemisch nach einem der Ansprüche 1 bis 6, wobei das Stoffgemisch ein Nahrungsergänzungsmittel, ein Nutrazeutikum, ein diätetisches und nährstoffhaltiges Stoffgemisch, ein Nahrungsmittel, ein Getränk, ein Nutrazeutikum, ein Medikament, ein medizinisches Nahrungsmittel oder ein Nahrungsmittel für besondere medizinische Zwecke ist.

8. Stoffgemisch zur Verwendung nach einem der Ansprüche 1 bis 7, wobei Hyperalgesie mit einer Krankheit, ausgewählt aus Osteoarthritis, Neuropathie, Kopfschmerzen, Migräne, Zystitis oder Prostatitis, assoziiert ist.

## Revendications

1. Composition comprenant ou consistant en un mélange de palmitoyléthanolamide, curcumine ou un extrait d'une plante appartenant au genre Curcuma, et pipérine ou un extrait d'une plante appartenant à la famille *Piperaceae* comprenant de la pipérine, pour une utilisation dans le traitement d'une hyperalgésie.

2. Composition pour une utilisation selon la revendication 1, laquelle composition est destinée à être utilisée par voie orale.

3. Composition pour une utilisation selon la revendication 1 ou 2, dans laquelle la quantité de curcumine est comprise entre 10 mg et 10 000 mg et/ou la quantité de palmitoyléthanolamide est comprise entre 10 et 5 000 mg.

4. Composition pour une utilisation selon l'une quelconque des revendications 1 à 3, dans laquelle la quantité de pipérine est comprise entre 0,01 et 500 mg.

5. Composition pour une utilisation selon l'une quelconque des revendications 1 à 4, sous une forme choisie parmi une gélule, une gélule molle, un comprimé, une pilule, une gélatine, une poudre, un granulé, une émulsion, une solution, un sirop.

6. Composition pour une utilisation selon l'une quelconque des revendications 1 à 5, comprenant en outre un ou plusieurs véhicules et/ou excipients.

7. Composition pour une utilisation selon l'une quelconque des revendications 1 à 6, laquelle composition est un complément alimentaire, un nutraceutique, une composition alimentaire et nutritionnelle, une denrée alimentaire, une boisson, un nutraceutique, un médicament, un aliment médicamenteux, ou un aliment à des fins médicales particulières.

8. Composition pour une utilisation selon l'une quelconque des revendications 1 à 7, dans laquelle l'hyperalgésie est associée à une maladie choisie parmi une arthrose, une neuropathie, une céphalée, une migraine, une cystite ou une prostatite.
